# EUROPEAN PATENT APPLICATION

(11) **EP 3 646 911 A1**
(43) Date of publication of application: **06.05.2020**
(21) Application number: 18823191.4
(22) Date of filing: 18.04.2018
(51) Int. Cl.: A61M 5/32, A61M 5/28

(54) **SYRINGE**

(30) Priority: 27.06.2017 JP 2017125659
(71) Applicant: Taisei Kako Co., Ltd., Kita-ku Osaka-shi Osaka 531-0072 (JP)
(72) Inventor: OGAWA, Yukihiro, Ibaraki-shi Osaka 567-0054 (JP); TANAKA, Yuji, Ayase-shi Kanagawa 252-1107 (JP)
(74) Representative: Sajda, Wolf E.
(86) International application number: PCT/JP2018/016024
(87) International publication number: WO 2019/003598

(57) **Abstract**

In a syringe (1) that allows an administration operation while a coil spring (16) is held by a spring holder (17) in a compressed state and a further thrust operation of the syringe barrel (2) after completion of administration releases the coil spring (16) from the hold of the spring holder (17), the coil spring (16) is prevented from being released from the hold of by the spring holder (17) inadvertently during administration. When, upon completion of administration of the liquid drug, the syringe barrel (2) is thrust toward the distal end relative to the cover (6), the spring holder (17) is configured to be movable from a hooked position toward the distal end relative to the cover (6), where the hook members (17b) are hooked on the cover (6) when in the hooked position, and a guide surface (S) is provided on the inner periphery of the cover (6) adapted, when the spring holder (17) has moved from the hooked position toward the distal end, to cause the hook members (17b) to radially deform such that the hook members cease to be hooked on the cover (6).

## Description

### TECHNICAL FIELD

The present invention relates to a syringe.

### BACKGROUND ART

To prevent a patient, when injecting a biomedical anticancer drug, an antirheumatic drug or other drugs at home, from accidentally hurting himself/herself with the needle, the applicant of the present application has been developing disposable syringes whose needle can be placed within the body until injection and, after injection, can be retracted back into the body, and has disclosed results of this effort in Patent Documents 1 and 2 listed below.

### PRIOR ART DOCUMENTS

### PATENT DOCUMENTS

Japanese Patent Document 1: JP 2014-212832 A
Japanese Patent Document 2: JP 2016-202438 A

In each of the syringes disclosed in Patent Documents 1 and 2, the injection needle is placed within a cover and a plunger during storage before use. During storage, the cover and plunger are arranged in the axial direction to abut each other to prevent the plunger from being thrust in relative to the cover.

During use, the cover and plunger are rotated relative to each other to reduce the total axial length of the cover and plunger such that the tip of the injection needle protrudes outward from the distal-end portion of the cover and, thereafter, the syringe barrel is thrust in relative to the cover and plunger to achieve administration of the liquid drug.

Further, the cover is composed of a first cover member (i.e., cylindrical body) and a second cover member (i.e., safety cover) that can be axially moved relative to each other by a small amount, where, upon completion of administration, the syringe barrel can be thrust yet more strongly to increase the total axial length of the first and second cover members.

When the total length of the first and second cover members has been increased to some extent, the hook members of the spring holder are broken to release the coil spring, and the resulting biasing force of the coil spring causes the cover to axially move relative to the injection needle further in the direction of the distal end such that the injection needle is retracted back into the cover.

### SUMMARY OF THE INVENTION

### PROBLEMS TO BE SOLVED BY THE INVENTION

A problem with the above-described conventional syringe construction is that, during administration, if the syringe barrel is momentarily thrust strongly, this strong thrust force is transmitted up to the hook members of the spring holder, potentially breaking the hook members even though administration is not yet completed.

### MEANS FOR SOLVING THE PROBLEMS

In connection with a syringe including:
a syringe barrel having an open distal-end portion and defining an interior space to be filled with a liquid drug;
a gasket fitted into the syringe barrel to seal in the liquid drug;
an injection needle having a base-end portion adapted to pierce through the gasket at least during administration of the liquid drug;
a plunger attached to the gasket and holding the injection needle;
a cover capable of being axially moved relative to the injection needle between a storage position and an administration position, wherein the cover contains therewithin a distal-end portion of the injection needle when in the storage position and the distal-end portion of the injection needle protrudes through a distal-end portion of the cover when the cover is in the administration position,
a coil spring adapted to bias the cover toward the storage position; and a spring holder adapted to hold the coil spring in a compressed state,
wherein the spring holder includes a hook member adapted to axially hook onto the cover, and
the hook member is configured in such a manner that the coil spring is held in the compressed state between the spring holder and the cover when the hook member is axially hooked on the cover, and, when, after completion of administration of the liquid drug, the syringe barrel is thrust in toward a distal end of the syringe relative to the cover, the hook member and the cover are unhooked such that the coil spring is released and biases the cover toward the storage position,
the present invention proposes a novel hook construction for the spring holder that prevents the coil spring from being released during administration of the liquid drug.

That is, the syringe of the present invention is characterized in that the spring holder is configured to become movable from a hooked position toward the distal end of the syringe relative to the cover, as the syringe barrel is thrust toward the distal end of the syringe relative to the cover after completion of administration of the liquid drug. The hook member is hooked on the cover in the hooked position.

Additionally, the cover has an inner periphery including a guide surface adapted, when the spring holder has moved from the hooked position toward the distal end of the syringe relative to the cover, to cause the hook member to radially deform such that the hook member ceases to be hooked on the cover.

In the syringe of the present invention described above, the thrust force during administration of a liquid drug does not act on the spring holder, and the hook member is not unhooked from the cover in an inadvertent manner. When, upon completion of administration of the liquid drug, the syringe barrel is thrust further toward the distal end, the spring holder slightly moves from the hooked position toward the distal end, and this movement causes the hook member to be guided on the guide surface and deformed radially; after this deformation, the hooked state of the hooked member is released such that the coil spring is released.

Thereafter, when the thrusting operation for the syringe barrel is halted, the coil spring, now released, biases the cover toward the storage position such that the injection needle is retracted back into the cover.

In the syringe of the present invention, the cover includes, in an integral manner, a cylindrical body adapted to be fitted around the syringe barrel and an inward flange extending radially inwardly from the distal-end portion of the cylindrical body, wherein the cylindrical body includes a support plane facing the distal end of the syringe, the spring holder is located adjacent to an inner periphery of the cylindrical body and rearward of the inward flange, a rear-end plane of the spring holder and a distal-end plane of the syringe barrel face each other in an axial direction so as to abut each other upon completion of administration of the liquid drug, the coil spring is located between the inward flange and the spring holder, the hook member is capable of hooking onto the support plane of the cylindrical body in a direction from the distal end of the syringe and deforming radially inwardly so as to cease to be hooked on the support plane of the cylindrical body.

In this construction, when the syringe barrel is further thrust after completion of administration of the liquid drug, the spring holder is pushed by the syringe barrel from the hooked position toward the distal end. Then, the hook member is guided on the guide surface while in abutment and deforms radially inwardly such that the hook member ceases to be hooked on the support plane and thus the coil spring is released.

### EFFECTS OF THE INVENTION

The syringe of the present invention represents a simple configuration that provides a novel engagement construction where the thrust force during administration of a liquid drug does not act on the hook member of the spring holder and the coil spring is not released in an inadvertent manner during administration.

### BRIEF DESCRIPTION OF THE DRAWINGS

- FIG. 1(a): illustrates operations of a syringe according to an embodiment of the present invention and shows a longitudinal cross-sectional view of the syringe as found during storage, i.e., in an initial state.
- FIG. 1(b): illustrates operations of a syringe according to an embodiment of the present invention and shows a longitudinal cross-sectional view of the syringe as found at the initiation of administration.
- FIG. 1(c): illustrates operations of a syringe according to an embodiment of the present invention and shows a longitudinal cross-sectional view of the syringe as found upon completion of administration.
- FIG. 1(d): illustrates operations of a syringe according to an embodiment of the present invention and shows a longitudinal cross-sectional view of the syringe as found in a needle retraction state after completion of administration.
- FIG. 2: shows an enlarged perspective view of the spring holder of the same syringe.

### EMBODIMENTS FOR CARRYING OUT THE INVENTION

Now, a preferred embodiment of the present invention will be described with reference to the drawings.

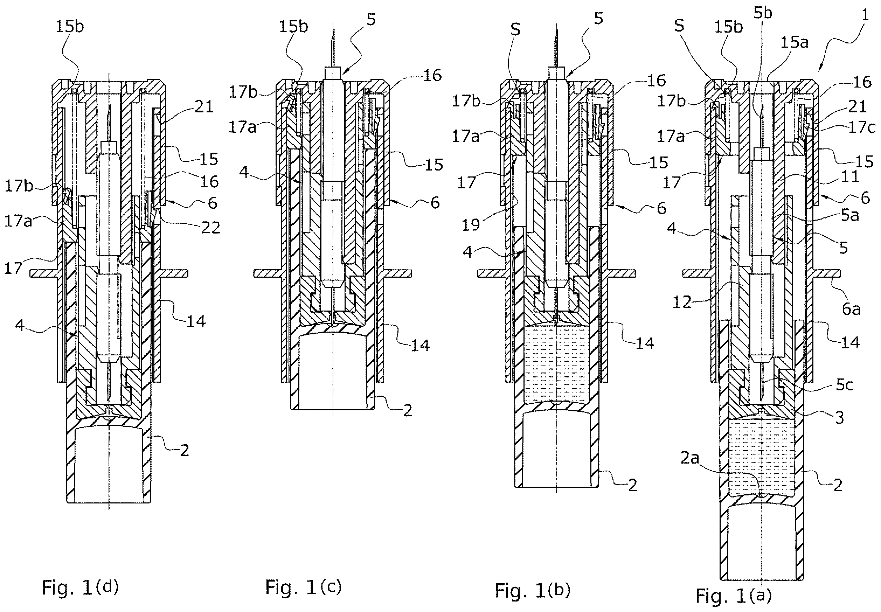

FIG. 1 shows cross-sectional views of a syringe 1 according to an embodiment of the present invention during an administration procedure; particularly, the left half of each cross-sectional view shows a longitudinal cross section of a portion of the syringe in which a hook member 17b of the spring holder 17 is present, while the right half shows a longitudinal cross section of a portion in which an elastic engagement member 17c of the spring holder 17 is present.

It is also noted that the syringe 1 of the present embodiment is substantially the same as the syringe disclosed by Patent Document 2 listed above, except for the hooking construction of the hook members of the spring holder; accordingly, the description and drawings of Patent Document 2 are incorporated herein by reference and the inventive syringe will be described using identical reference characters.

The syringe 1 of the present embodiment includes: a syringe barrel 2 shaped as a bottomed cylinder and made of transparent or translucent glass or synthetic resin; a gasket 3 sealingly introduced into the syringe barrel 2 through the distal-end opening so as to be movable in the axial direction; a plunger 4 for thrusting the gasket 3 toward the base end relative to the syringe barrel 2; a double-ended injection needle 5 attached to the plunger 4 so as to be movable in the axial direction; and a cover 6 covering the outer periphery of the injection needle 5, these components being positioned to be concentric. The gasket 3 is made of an elastic material, such as rubber.

The cover 6 includes a cylindrical first cover member 14 (i.e., cylindrical body) provided with an integral finger-hook flange 6a located on the outer periphery of an intermediate portion thereof as determined along the axial direction, and a second cover member 15 mounted on and fixed to the distal-end portion of the first cover member 14 in an integral manner. The syringe barrel 2 is inserted into the first cover member 14 through its base-end portion so as to be movable in the axial direction.

The second cover member 15 includes, in an integral manner, a cylindrical portion adapted to be fitted around the first cover member 14, and an inward flange 15b extending radially inwardly from the distal-end portion of the cylindrical portion; at the axis center of the inward flange 15b is provided a needle retraction hole 15a, through which the distal-end portion 5b of the injection needle 5 may be projected and retracted.

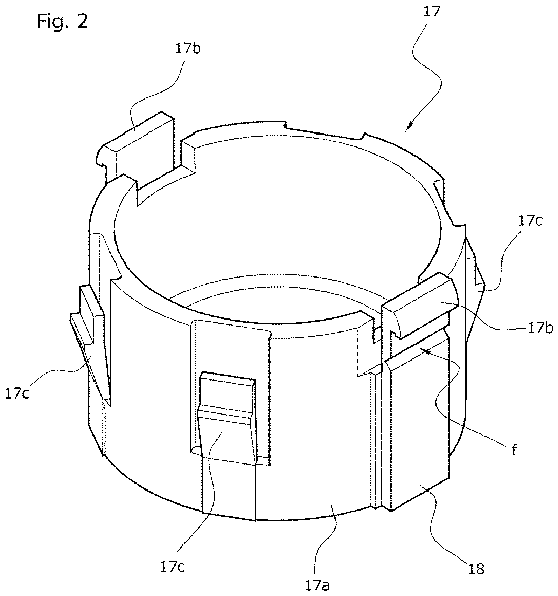

Inside the cover 6 are provided a coil spring 16 and a spring holder 17 for holding the spring 16 while keeping the spring axially compressed. As also shown in FIG. 2, the spring holder 17 includes a ring portion 17a positioned to face the upper plane of the syringe barrel 2 at the open distal-end portion (i.e., distal-end plane) such that the ring and the plane are arranged in the axial direction, and a hook member 17b for hooking onto the distal-end plane of the first cover member 14 (i.e., support plane) in the direction from the distal end.

A portion of the outer periphery of the hook member 17b associated with the distal end is shaped as an inclined surface to enable smooth guiding by a guide surface S, discussed below, thereby further ensuring that the hook member 17b deforms radially inwardly.

More particularly, a pair of hook members 17b are provided to face each other and be arranged across a diameter. Each hook member 17b is integral to the ring portion 17a with a bent portion f located in between, and includes a hook claw for hooking onto the distal-end plane of the first cover member 14 (i.e., support plane) in the direction from the distal end.

The spring holder 17 is constructed such that its ring portion 17a is positioned adjacent to the inner periphery of the first cover member 14 and rearward of the inward flange 15b, and is axially movable, relative to the cover 6 and by a small amount, from a hooked position (i.e., position of the spring holder 17 relative to the cover 6 as found when the hook members 17b are hooked on the distal-end plane of the first cover member 14) toward the distal end.

The rear-end plane of the ring portion 17a of the spring holder 17 and the distal-end plane of the syringe barrel 2 face each other and are arranged in the axial direction, so as to abut each other upon completion of administration of the liquid drug, as shown in FIG. 1(c). The coil spring 16 is located between the inward flange 15b and ring portion 17a.

Upon completion of administration of the liquid drug, i.e., when the gasket 3 has just contacted the bottom surface of the syringe barrel 2, the hook members 17b of the spring holder 17 are still in engagement with the distal-end plane of the first cover member 14, and the distal-end plane of the syringe barrel 2 and the rear-end plane of the ring portion 17a of the spring holder 17 are in abutment with each other.

Upon completion of administration of the liquid drug, when the syringe barrel 2 is strongly thrust further toward the distal end relative to the cover 6 against the biasing force of the coil spring 16, the gasket 3 slightly elastically deforms, and thus the spring holder 17 is moved by the syringe barrel 2 slightly in the axial direction toward the distal end relative to the cover 6, as shown in FIG. 1(c).

At this moment, the hook members 17b of the spring holder 17 are guided radially inwardly by a tapered guide surface S provided on the perimeter of the rear side of the inward flange 15b such that the hook members 17b deform radially inwardly so as to cease to be hooked on the distal-end plane of the first cover member 14; the hook members 17b are unhooked from the distal-end plane of the first cover member 14 such that the coil spring 16 is released.

Then, when the thrusting operation for the syringe barrel 2 is halted, the biasing force of the coil spring 16 causes the cover 6 to axially move toward the distal end relative to the syringe barrel 2, as shown in FIG. 1(d), such that the distal-end portion 5b of the injection needle 5 is contained within the cover 6.

As shown in FIG. 2, a protrusion 18 is provided at a predetermined circumferential position on the outer periphery of the ring portion 17a (i.e., the same position as each hook member 17b in the shown implementation), and a guide groove 19 is provided on the inner periphery of the first cover member 14 to extend in the axial direction to allow the associated protrusion 18 to slide thereon in the axial direction such that the spring holder 17 is held in the cover 6 so as to be capable of axially moving and incapable of rotating relative to the cover.

On the inner periphery of the first cover member 14 are further provided, as also shown in Patent Document 2, a plurality of engagement grooves (indicated by number 20 in Patent Document 2) that extend in the axial direction and are located at predetermined circumferential positions, where a first engagement recess 21 and a second engagement recess 22 are provided in each engagement groove to be axially separated from each other.

Each of the engagement recesses 21 and 22 may be constituted by a through-hole. On the outer periphery of the ring portion 17a is provided an elastic engagement member 17c to be positioned in a first engagement recess 21. A plurality of engagement members 17c are provided to be circumferentially arranged and correspond to the engagement grooves.

Each of the engagement members 17c has a base end that is connected to the base-end portion of the ring portion 17a, as well as a distal-end portion that is radially expandable and contractable. A stepped portion for engagement is provided on the distal-end portion of the engagement member 17c, and the outer periphery of the engagement member 17c is an inclined surface that gradually decreases in diameter toward the distal end.

When the stepped portions for engagement of the engagement members 17c engage those edges of the engagement recesses 21 and 22 which are located closer to the distal end, this prevents the first cover member 14 from moving toward the base end relative to the ring portion 17a; on the other hand, when the ring portion 17a moves toward the base end relative to the first cover member 14, the guiding by the inclined surface causes the engagement members 17c elastically deform radially inwardly, allowing the spring holder 17 to slide toward the base end relative to the first cover member 14 where the engagement members 17c are guided by the engagement grooves.

However, a movement of the ring member 17a relative to the first cover member 14 further toward the base end than the position taken when the engagement members 17c are in engagement with the second engagement recesses 22 is prevented by the syringe barrel 2, gasket 3, plunger 4 and second cover member 15.

The syringe barrel 2 is filled in advance with a predetermined amount of a liquid drug in an aseptic room, and the gasket 3 is sealingly introduced into the syringe barrel 2 so as to seal in the liquid drug. A small indentation 2a is provided at the center of the bottom wall of the syringe barrel 2.

While the syringe disclosed in Patent Document 1 includes an end cap attached to the base-end portion of the syringe barrel, an end cap is omitted in the syringe of the present embodiment in order to reduce the number of components, where the base-end portion itself of the syringe barrel extends toward the axis . Further, while the syringe disclosed in Patent Document 1 includes a stopper attached to the open distal-end portion of the syringe barrel, such a stopper is also omitted according to the present embodiment.

The injection needle 5 includes a columnar needle base 5a and a needle tube extending through the needle base 5a in the axial direction. The needle tube protrudes from both ends, as determined along the axial direction, of the needle base 5a, where the portion protruding toward the distal end constitutes the distal-end needle portion 5b, while the portion protruding toward the base end constitutes the base-end needle portion 5c. A sharply cut edge is provided on the tip of each of the needle portions 5b and 5c.

The gasket 3 is hermetically fitted around the base-end portion of the plunger 4. Further, the axis-center portion of the gasket 3 has a smaller wall thickness so as to be easily pierced through by the base-end portion 5c of the injection needle 5. As shown in FIG. 1(a), during storage before administration, the base-end portion 5c of the injection needle 5 is separated from the gasket 3, located further toward the distal end in the axial direction.

The plunger 4 is shaped as a hollow cylinder with a bore extending therethrough in the axial direction, and the injection needle 5 is held by this plunger 4.

The plunger 4, injection needle 5, and cover 6 constitute a safe operation mechanism for preventing an inadvertent or careless operation to prevent an accident involving the injection needle 5. For details about this safe operation mechanism, see description and drawings of Patent Document 2 listed above. The following is some of the main points of the safe operation mechanism cited from Patent Document 2.

The mechanism includes a pair of first elastic engagement members 11 integral with the cover 6 and that can be fitted around the needle base 5a in the direction from the distal end for holding the injection needle 5, and a pair of second elastic engagement members 12 integral with the plunger 4 and that can be fitted around the needle base 5a in the direction from the base end for holding the injection needle 5.

Each of the elastic engagement members 11 and 12 is shaped as a column with an arc-shaped transverse cross section. The pair of first elastic engagement members 11 are separated in the circumferential direction and, particularly, positioned to be opposite across a diameter according to the present embodiment. Similarly, the pair of second elastic engagement members 12 are separated in the circumferential direction and positioned to be opposite across a diameter.

During storage of the syringe 1, these first and second elastic engagement members 11 and 12 are arranged in the axial direction, and the base-end surface of each of the first elastic engagement members 11 and the distal-end surface of the corresponding one of the second elastic engagement members 12 are placed to abut each other and are thus locked in an extended state where the total length of the first and second elastic engagement members 11 and 12 is larger than the total length of the injection needle 5, thereby preventing the plunger 4 from being thrust toward the distal end relative to the cover 6 in the locked position. In this extended state, the distal-end portion 5b of the injection needle 5 is placed within the cover 6.

On the other hand, when the cover 6 is rotated relative to the plunger 4 by 90°, the plurality of second elastic engagement members 12 face the plurality of spaces defined by the first elastic engagement members 11 such that the first and second elastic engagement members 11 and 12 are arranged alternately in the circumferential direction; by thrusting the plunger 4 toward the distal end relative to the cover 6 in this unlocked position, the plurality of first elastic engagement members 11 alternately engage the plurality of second elastic engagement members 12, thereby reducing the total length of the cover 6 and plunger 4.

When the total axial length of the cover 6 and plunger 4 is thus reduced, the base-end portion 5c of the injection needle 5 pierces through the gasket 3 and the distal-end needle portion 5b protrudes in the direction of the distal end through the needle retraction hole 15a of the cover 6.

The first elastic engagement members 11 are integral with the inward flange 15b of the second cover member 15 of the cover 6 and extends from the back side of the inward flange 15b toward the base end (i.e., downward in the drawings), and the elastic engagement members 11 are elastically deformable such that their base-end portions (i.e., bottom ends) are expanded in diameter.

On the other hand, the second elastic engagement members 12 extend from an intermediate portion, as determined along the axial direction, of the plunger 4 toward the distal end (i.e., upward), and elastically deformable such that their distal-end portions (i.e., top ends) are expanded in diameter. The first elastic engagement members 11 have a larger axial length than the second elastic engagement members 12.

The needle base 5a of the injection needle 5 includes a first shaft portion adjacent to the distal end around which the first elastic engagement members 11 are fitted during storage, a second shaft portion adjacent to the base end around which the second elastic engagement members 12 are fitted, and a small-diameter shaft portion provided between the first and second shaft portions.

On the outer periphery of the first shaft portion are provided: a pair of restriction ribs arranged opposite in a diametrical direction for abutting edges, as determined along the circumferential direction, of the first elastic engagement members 11 so as to allow the first elastic engagement members 11 to rotate up to about 90° relative to the first shaft portion but prevent them from rotating relative to it more than about 90°; and a pair of grooves arranged opposite in a diametrical direction and extending across the entire axial length of the first shaft portion.

On the outer periphery of the second shaft portion are provided: four restriction ribs at different circumferential positions for abutting edges, as determined along the circumferential direction, of the second elastic engagement members 12 so as to prevent the second elastic engagement members 12 from rotating relative to the second shaft portion; and a pair of grooves arranged opposite in a diametrical direction and extending across the entire axial length of the second shaft portion.

These two pairs of grooves are positioned to be displaced in the circumferential direction. Each of the first elastic engagement members 11 includes a key constituted by a protrusion is provided on the inner surface of the base-end portion, and each of the second elastic engagement members 12 includes a key constituted by a protrusion provided on the inner surface of the distal-end portion.

In the initial state for storage, the keys are located around the small-diameter shaft portion, where the keys on the first elastic engagement members 11 engage, in the axial direction, a step between the small-diameter shaft portion and first shaft portion, while the keys on the second elastic engagement members 12 engage, in the axial direction, a step between the small-diameter shaft portion and second shaft portion, thereby preventing the injection needle 5 from moving in the axial direction relative to the first and second elastic engagement members 11 and 12.

During storage, the keys on the second elastic engagement members 12 are already positioned to face the grooves on the first shaft portion in the axial direction and, when the first elastic engagement members 11 are rotated about 90°, the second elastic engagement members 12 become movable relative to the injection needle 5 toward the distal end, where the keys move within the grooves in the axial direction. On the other hand, during storage, the keys on the first elastic engagement members 11 are located about 90° relative to the grooves on the second shaft portion.

When the first elastic engagement members 11 are rotated about 90° relative to the injection needle 5, the keys are positioned to face the grooves in the axial direction such that the injection needle 5 becomes movable relative to the first elastic engagement members 11 toward the distal end, where the keys move within the grooves in the axial direction.

Further, when, starting with the initial state, the cover 6 is rotated about 90° relative to the injection needle 2 and thrust in, the first elastic engagement members 11 are thrust relative to the injection needle 5 in the axial direction such that the distal-end portion 5b of the injection needle 5 protrudes from the needle retraction hole 15a of the cover 6; at this time, the distal-end portions of the restriction ribs on the first shaft portions abut the top plate of the cover 6 in the axial direction to surround the needle retraction hole 15a, thereby making it possible to force the injection needle 5 to be thrust toward the gasket 3 toward the base end.

To prevent the cover 6 from being rotated inadvertently relative to the plunger 4 from the initial state for storage, the needle base 5a of the injection needle 5 includes a protrusion provided on the outer periphery of the small-diameter shaft portion.

When the cover 6 rotates from the locked position to the unlocked position, each first elastic engagement member 11 is slightly deformed to be radially expanded and a key climbs over the protrusion. The resistance to the rotating operation represented by this prevents the cover 6 from being inadvertently rotated.

Further, according to the present embodiment, a cylindrical deformation-prevention part is formed integrally with the plunger. This deformation-prevention part is located closer to the distal end than the second elastic engagement members 12 are, and are fitted around the plurality of first elastic engagement members 11 in the initial state for storage to prevent the first elastic engagement members 11 from being deformed to be radially expanded, thereby preventing the keys on the first elastic engagement members 11 from being disengaged from the relevant step on the injection needle 5.

Since the deformation-prevention part is cylindrical in shape, the deformation prevention part constantly prevents the first elastic engagement members 11 from being deformed to be radially expanded even when the pair of first elastic engagement members 11 rotate relative to the deformation-prevention part. The inner diameter of the deformation-prevention part is slightly larger than the outer diameter of the pair of first elastic engagement members 11 to permit the first elastic engagement members 11 to be slightly deformed to be radially expanded so they can climb over the protrusion.

When the plunger 4 is thrust toward the distal end relative to the cover 6, the distal-end portion of the deformation-prevention part abuts the inward flange 15b of the cover 6 such that the second elastic engagement members 12 are spaced apart from the inward flange 15b by the axial length of the deformation-prevention part. According to the present embodiment, a pair of holding members having an axial length substantially equal to that of the deformation-prevention part extend, between the pair of first elastic engagement members 11, from the inward flange 15b toward the base end in the axial direction.

Each of these holding members has a transverse cross section that is substantially the same as that of the first elastic engagement members 11, and the holding members and first elastic engagement members 11 are arranged alternately so as to be slightly separated in the circumferential direction. The above-discussed spaces for the engagement with the second elastic engagement members 12 are provided adjacent to the base-end portions of the holding members.

When the first and second elastic engagement members 11 and 12 are in the extended state, the holding members are fitted around the outer periphery of the distal-end portion of the needle base 5a, but are located closer to the distal end than the distal-end portions of the restriction ribs are, thereby allowing the cover 6 to be rotated relative to the injection needle 5. At this moment, the injection needle 5 is supported by the cover 6 at four points, i.e., at the two first elastic engagement members 11 and two holding members, thereby stabilizing the injection needle 5 in its held position.

On the other hand, when the cover 6 is rotated from the initial state for storage by 90° and then the plunger 4 is thrust in relative to the cover 6, the injection needle 5 moves in the axial direction relative to the cover 6 toward the distal end, and the restriction ribs slide in the axial direction within the slits between the holding members and first elastic engagement members 11. At this time, in addition to the four-point support, the engagement of the restriction ribs and the slits further stabilizes the injection needle 5 in its held position to achieve a yet smoother operation for causing the injection needle 5 to protrude.

Further, the outer periphery of the deformation prevention part is covered with the first cover member 14 of the cover 6. A pair of recesses are provided that occupy a pair of opposite portions of the deformation prevention part arranged across a diameter and accounting for some of the axial dimension of the deformation prevention part, each recess extending through in a direction perpendicular to the direction of that diameter. Preferably, each recess may be constituted by edges, as determined along the circumferential direction, of a through-hole extending through the deformation prevention part in a radial direction, the edges being in a flat plane extending in that perpendicular direction.

On the first cover member 14 is provided a peephole that, when the cover is in the locked position, faces a recess in the hole's direction of through-extension and, when the cover is in the unlocked position, faces the deformation prevention part. A total of four peepholes are provided, one located on each of the sides of each of the two recesses as determined along the recess's direction of through-extension.

If the plunger 4 is in a color that is different from those of the other components and thus is conspicuous, e.g., red, the red portions are hardly visible in the peepholes when the cover 6 is in the locked position, and the peepholes are filled with red when the cover 6 is rotated to the unlocked position. Thus, the deformation prevention part functions as an indicator that allows the current state of the cover 6 to be quickly determined by visual inspection.

The present invention is not limited to the above-described embodiment, and can be modified in design as appropriate.

## Claims

1. A syringe comprising:
a syringe barrel having an open distal-end portion and defining an interior space to be filled with a liquid drug;
a gasket fitted into the syringe barrel to seal in the liquid drug;
an injection needle having a base-end portion adapted to pierce through the gasket at least during administration of the liquid drug;
a plunger attached to the gasket and holding the injection needle;
a cover capable of being axially moved relative to the injection needle between a storage position and an administration position, wherein the cover contains therewithin a distal-end portion of the injection needle when in the storage position and the distal-end portion of the injection needle protrudes through a distal-end portion of the cover when the cover is in the administration position,
a coil spring adapted to bias the cover toward the storage position; and
a spring holder adapted to hold the coil spring in a compressed state,
wherein the spring holder includes a hook member adapted to axially hook onto the cover, and
wherein the hook member is configured in such a manner that the coil spring is held in the compressed state between the spring holder and the cover when the hook member is axially hooked on the cover, and, when, after completion of administration of the liquid drug, the syringe barrel is thrust in toward a distal end of the syringe relative to the cover, the hook member and the cover are unhooked such that the coil spring is released and biases the cover toward the storage position,
**characterized in that**
the spring holder is configured to, as the syringe barrel is thrust toward the distal end of the syringe relative to the cover after completion of administration of the liquid drug, become movable from a hooked position, in which the hook member is hooked on the cover, toward the distal end of the syringe relative to the cover, and
the cover has an inner periphery including a guide surface adapted, when the spring holder has moved from the hook position toward the distal end of the syringe relative to the cover, to cause the hook member to radially deform such that the hook member ceases to be hooked on the cover.

2. The syringe according to claim 1,
wherein the cover includes, in an integral manner, a cylindrical body adapted to be fitted around the syringe barrel and an inward flange extending radially inwardly from the distal-end portion of the cylindrical body, wherein the cylindrical body includes a support plane facing the distal end of the syringe, the spring holder is located adjacent to an inner periphery of the cylindrical body and rearward of the inward flange, a rear-end plane of the spring holder and a distal-end plane of the syringe barrel face each other in an axial direction so as to abut each other upon completion of administration of the liquid drug, the coil spring is located between the inward flange and the spring holder, the hook member is capable of hooking onto the support plane of the cylindrical body in a direction from the distal end of the syringe and deforming radially inwardly so as to cease to be hooked on the support plane of the cylindrical body.
